# EUROPEAN PATENT SPECIFICATION

(11) **EP 3 032 947 B1**
(45) Date of publication and mention of the grant of the patent: **04.07.2018**
(21) Application number: 13750564.0
(22) Date of filing: 16.08.2013
(51) Int. Cl.: A61K 47/10, A01N 25/02, A01N 25/06, A01N 37/02, A01N 31/02, A61K 9/00, A61K 31/047, A61K 31/19, A61K 33/20, A01P 1/00

(54) **ANTIMICROBIAL SKIN CARE LIQUID COMPOSITIONS FORMULATED FOR USE IN COLD TEMPERATURE CONDITIONS**
ANTIMIKROBIELLE HAUTPFLEGE-FLÜSSIGKEITSZUSAMMENSETZUNGEN ZUR VERWENDUNG BEI KALTEN TEMPERATUREN
COMPOSITIONS LIQUIDES ANTIMICROBIENNES POUR LE SOIN DE LA PEAU FORMULÉES POUR ÊTRE UTILISÉES DANS DES CONDITIONS DE BASSE TEMPÉRATURE

(43) Date of publication of application: 22.06.2016
(73) Proprietor: Ecolab USA Inc., St. Paul, MN 55102 (US)
(72) Inventor: KLEINE, Tillmann, 50668 Köln (DE); KILLEEN, Jonathan Scott, 68169 Mannheim (DE); BREIDERHOFF, Helen, 41334 Nettetal (DE)
(74) Representative: Michalski Hüttermann & Partner Patentanwälte mbB
(86) International application number: PCT/EP2013/067177
(87) International publication number: WO 2015/022033

(56) References cited:
- EP-A1- 0 906 724
- WO-A1-00/13507
- WO-A2-02/28180
- US-A1- 2009 247 485

## Description

### Field of the Invention

The present invention is directed to antimicrobial skin care liquid compositions having specialized benefit of skin protection and conditioning for the control of mastitis in milk producing animals that is especially useful in cold temperature environments and a method of treating for mastitis in cold temperature conditions. Furthermore, in some embodiments, the invention provides mastitis treatment liquid compositions and formulations having specialized benefit of skin protection and conditioning on the teats of an animal that is already in poor health.

### Background of the Invention

Mastitis is an inflammation of the mammary gland. Bovine mastitis is the most common and most costly disease affecting dairy herds. Some estimates suggest at least half of the dairy animal population have some degree or form of mastitis. This condition results in lowered milk yield and reduced milk quality.

Economic loss to mastitis in the U. S. is estimated at $1.8 billion or approximately 10% of total milk sales with two-thirds of this loss due to reduced milk production from infected cows. In dairy cattle, mastitis typically results from microorganisms; usually bacteria, that invade the udder, multiply in the milk producing tissues, and synthesize toxins, a byproduct of bacterial metabolism.

The characteristic features of inflammation are swelling, heat, redness, pain and disturbed function.

While the animal immune system can fight intramammary infections, many chronic infections remain sub-clinical (asymptomatic) and undetected unless diagnosed by laboratory testing. Sub-clinical mastitis can result in a reservoir of microorganisms, which can lead to the infection of other animals within the herd.

More than 80 species of microorganisms have been identified as causal agents, although approximately 95% of mastitis is caused by four pathogens:
*Staphylococcus aureus*, *Streptococcus agalactiae*, *Streptococcus dysagalactiae*, and *Streptococcus uberis.* Mastitis causing pathogens fall into two categories namely contagious and environmental. Contagious bacteria, such as *Streptococcus agalactiae* and *Staphylococcus aureus*, primarily colonize host tissue sites such as mammary glands, teat canals, teat skin lesions etc. and are spread from one infected cow to another during the milking process. Environmental bacteria, often streptococci, enterococci and coliform organisms, are commonly present within the cow's surroundings from sources such as cow feces, soil, plant material, bedding or water, and infect by casual opportunistic contact with an animal. This distinction, although not exclusive, is of practical importance because different dairy herd maintenance measures are needed for the different groups of microorganisms. In all bovine mastitis cases, whatever the causal microorganism, the route of transmission of the invading pathogen into the inner gland of the udder is through the teat orifice and teat canal.

Management of dairy herds focuses attention on treatment of both established mastitis and on prevention of new intramammary infections. Therapy and hygiene are two fundamental components of an effective mastitis control program. Each is applied in concert, and each operates independently. The primary effect of therapy is to eliminate established infections, whereas, hygiene reduces the incidence of new infection by interrupting transmission vectors. A non-exhaustive list of ancillary factors which may be employed for the elimination and prevention of mastitis, include, post-lactation antibiotic infusion into the udder (e. g., dry cow treatment); and, post-milking teat antisepsis or "teat dipping" during lactation.

Researchers agree, and an abundance of published evidence supports the concept, that dipping teats into an effective antimicrobial solution immediately after each milking is the single most effective procedure for decreasing new intramammary infections in lactating cows. The efficacy and value of teat dipping has since been confirmed in dozens of field trials, and it is now accepted that an effective teat dip can reduce the incidence of new intramammary infections at least 50% and often up to 90%.

To reduce mastitis, commercial teat dips have been developed containing a variety of antimicrobial agents including iodophors, quaternary ammonium compounds, chlorhexidine salts, chlorine release compounds (e. g. alkali hypochlorites), oxidizing compounds (e. g. hydrogen peroxide, peracids), protonated carboxylic acids (e. g. octanoic, nonanoic, decanoic, acids), acid anionics (e. g. alkylaryl sulfonic acids), and chlorine dioxide (from chlorite). These agents, which have varying degrees of effectiveness, limit the transmission of mastitis by reducing pathogen populations on the teat.

Teat dips, can also be divided into two broad classifications. The Class I type are antimicrobial and are applied to kill microorganisms already present in the teat canal or on the surface of the teat skin. By design, their microbiological effect is immediate and they primarily target the contagious organisms that are vectored between animals during the premilking, milking and post-milking process.

The Class II type teat dip, often referred to as a "teat sealer", is a film-forming or coating composition which may or may not be antimicrobial; and, functions by developing a residual protective barrier on the teat thus providing prophylaxis by sealing the teat orifice from environmental contamination. The film, which forms on the surface of the teat, serves as a physical barrier through which mastitis causing pathogens cannot penetrate during the intermilking period.

General disclosures of teat dip technology and publications are shown in, for example, WO 02/28180 A2.

EP 0906 724 A1 refers to a mastitis control teat dip composition provides rapid initial kill, a useful highly pseudoplastic rheology, a barrier/film-forming capacity, a unique antimicrobial composition that is stable over an extended period of time, and unexpected long term microbial control.

WO 00/13507 A1 refers to a stabilized two-part disinfecting system comprising a first part and a second part adapted to be mixed to yield an aqueous disinfecting composition. The first part contains a non-esterifying acid and an alcohol- containing humectant or antifreeze, while the second part contains a salt of an organic acid and an optional metal chlorite.

US 2009/247485 A1 refers to a long-lasting persistent, uniform, film-forming skin protecting compositions provide long-lasting persistent barrier films when applied to skin. The compositions have particular utility as barrier teat dips for protecting cows against mastitis and as wound care agents.

There is a growing acceptance among academics, veterinarians and dairy herd management that proactive maintenance of teat health and skin condition is an integral part of a complete program for the prevention, control and remedial correction of mastitis in mild producing animals. The assertion is that a healthy milk delivery organ can naturally retard and more readily withstand the adverse affects of infection.

Although many teat dip products are available, and there remains a continuing need for new and effective teat dip liquid compositions having immediate and long lasting antimicrobial effect against a wide spectrum of mastitis causing organisms; there is need for such antimicrobial liquid compositions which additionally provide superior skin care conditioning and health maintenance functions.

Additionally, many teat dip products are not conducive for use in cold environment conditions.

Moreover, known teat dip products are not conducive for use as spray in cold environment conditions for the reasons given below.

For example, many teat dip compositions include a large amount of water, and can freeze in cold environments and/or contain glycol that leads to a viscosity at cold temperature beeing too high for spray applications.

Such compositions can freeze, leads to an insufficient spray cone angle and spray pattern, and cause irritation, chapping and frostbite. These conditions can enhance the incidence of mastitis caused by opportunistic invasive microorganisms.

There is a continuing need for new and effective teat dip liquid compositions, having immediate and long lasting antimicrobial effect against a wide spectrum of mastitis causing organisms that are formulated for use, for example as a spray, in cold environments.

### Summary of the Invention

The present invention is directed to novel antimicrobial and skin care liquid compositions according to claim 1, which can be used as a teat dip for milk producing animals. Thus, the embodiments of this invention provide both superior antimicrobial protection against mastitis causing organisms and skin conditioning for maintenance of healthy teats; and in addition provide protection against the adverse affects of frigid weather, one of the major causes of irritation, chapping and frostbite, which enhance the incidence of mastitis caused by opportunistic invasive microorganisms.

Still further benefit is accomplished by spray application of at least some preferred antimicrobial and skin care liquid compositions of this invention providing at cold temperature an excellent spray cone angle and spray pattern on teats.

Providing antimicrobial and skin care liquid compositions of this invention having excellent spray cone angle and spray pattern on teats at cold temperatures are important, for treating and/or avoiding poor health condition, for example to facilitate and accelerate disinfection, faster repair and/or the healing process of teats.

It will be noted that at several places throughout the Specification, guidance is provided through lists of examples. In each instance, the recited lists serve only as representative groups. It is not meant, however, that the lists are exclusive.

In preferred embodiments of a teat dip liquid compositions, the teat dip formulation also may includes significantly high concentrations of one or more occlusive agents which provide a protective barrier on the teat surface against irritating physical abrasion as from the mechanical action of the milking equipment or tissue damaging or disruptive environmental conditions such as frigid temperatures, wind chill, dehydration, windburn and sunburn. These agents may be, as example, but not meant to be inclusive: surfactants, emollients, lubricants, humectants, moisturizers, solvents etc. or mixtures thereof having the purpose of generally protecting and conditioning the teat skin surfaces thereby promoting healthy milk producing animals.

A teat dip liquid compositions can also include or be free of: a rheology modifier, a film-forming agent or admixture, a buffer system, a hydrotrope or coupler, or solvent, an emollient, skin conditioner or a lubricant, surfactant, color marker, fragrance, anti-irritants and healing agents, antioxidants, UV absorbers, vitamins or admixtures thereof.

In general, the invention also includes a method of controlling mastitis in milk producing animals comprising applying, in particular spraying, the liquid compositions to a teat of an animal.

In general, the invention also includes a method of controlling mastitis in milk producing animals comprising applying the liquid compositions to a teat of an animal.

The liquid compositions of the invention are particularly beneficial when applied in colder environmental temperatures, for example at temperatures of ≤ 0° C. The liquid compositions of the invention maintain its sprayability at very low temperatures at -8° C, preferably at -12° C, more preferred -15° C and/or most preferred at -20° C.

In some preferred embodiments the liquid compositions of the invention maintain its sprayability at very low temperatures at -8° C, preferably -12° C, more preferred -15° C and/or most preferred at -20° C.

### Detailed Description of the Invention

The present invention is directed to antimicrobial skin care liquid compositions, useful in cold temperature environments, comprising:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C₂ to C₆ organic monoalcohol selected from the group comprising ethanol, n-propanol, isopropanol, n-butanol, sec-butanol, iso-butanol, tert-butanol, pentanol and/or hexanol;
- ≥ 0.1 wt.-% to ≤ 10 wt.-% of sorbitol; and
- ≥ 0.1 wt.-% to ≤ 15 wt.-% of at least one C2 to C6 organic acid; wherein
the weight-% of the components are based on the total weight amount of the antimicrobial skin care liquid composition; wherein the composition has a viscosity at -8° C, and/or at -20° C of ≥ 1 mPas to ≤ 35 mPas, measured with a Brookfield viscometer, using a spindle 61 at 12 rpm..

The components are selected such that the total weight amount of all components of the antimicrobial skin care liquid composition is 100 wt.-%.

The antimicrobial skin care liquid composition of the invention can be preferably a teat dip antimicrobial skin care liquid composition.

It has been surprisingly found, that a selected mixture of monoalcohol, diolalcohol, trialcohol and sugar alcohol for a defined concentration range provides an excellent deep temperature spaying performance, such as spray cone angle and spray pattern, at temperatures up to -8° C, preferably -12° C, more preferred -15° C and/or most preferred at -20° C.

An antimicrobial skin care liquid compositions according to the invention may have a viscosity at -8° C, preferably -12° C, more preferred -15° C and/or most preferred at -20° C of ≥ 1 mPas to ≤ 35 mPas, preferably ≤ 30 mPas, more preferred ≤ 25 mPas, and most preferred ≤ 20 mPas or ≤ 15 mPas or ≤ 10 mPas or ≤ 5 mPas, measured with a Brookfield viscometer, using a spindle 61 at 12 rpm.

The antimicrobial skin care liquid compositions of the invention can be advantageously used as a teat dip for control of mastitis in milk producing animals. The compositions of the invention are formulated for use in cold temperature environments, for example in environments having a temperature of ≤ 0° C, such as ≥ -20° C to ≤ 0° C, for examples at very low temperatures at ≤ -10° C, ≤ -15° C or below.

According to one aspect of the present invention antimicrobial skin care liquid compositions maybe preferred they don't freeze at ≤ -15° C.

However, formulating of the invention can also be used in warm environment conditions. Some embodiments are particularly useful to facilitate faster repair of teats that are already in poor health regardless of the temperature. For example, some embodiments are particularly beneficial on chapped or irritated teats.

One improvement of the antimicrobial skin care liquid compositions of the invention is that the composition has excellent spaying performance, such as spray cone angle and spray pattern, at temperatures up to -20° C.

The spray cone angle can be at -8° C, preferably -12° C, more preferred -15° C and/or most preferred at -20° C of ≥ 10° to ≤ 180°, preferably of ≥ 15° to ≤ 160°, more preferred of ≥ 20° to ≤ 120°, in addition preferred of ≥ 30° to ≤ 100°, or of ≥ 40° to ≤ 90°, or of ≥ 50° to ≤ 80°. However, best results with respect to spray cone angle and spray pattern are achieved of ≥ 60° to ≤ 150° at ≤ -12° C with antimicrobial skin care liquid compositions according to the invention having a viscosity of < 20 mPas, preferably ≤ 15 mPas, and more preferred ≤ 10 mPas, measured with a Brookfield viscometer, using a spindle 61 at 12 rpm.

According to one aspect of the invention the weight amounts of at least one C₃ to C₆ organic triolalcohol, of at least one C₂ to C₆ organic diolalcohol, of at least one C₂ to C₆ organic monoalcohol, of at least one HOCH₂[CH(OH)]ₙCH₂OH sugar alcohol with n = 2 to 6; and of at least one C2 to C6 organic acid, are ≥ 20 wt.-% and ≤ 80 wt.-%, preferably ≥ 25 wt.-% and ≤ 60 wt.-%, and more preferred ≥ 30 wt.-% and ≤ 50 wt.-%, based on the total weight amount of the antimicrobial skin care liquid composition.

### Definition of terms

So that the invention maybe more readily understood, certain terms are first defined.

As used herein, "weight percent", "wt-%", "percent by weight", "% by weight", and variations thereof refer to a composition, component, substance or agent as the weight of that composition, component, substance or agent of the antimicrobial skin care liquid composition divided by the total weight of the antimicrobial skin care liquid composition or use composition and multiplied by 100. It is understood that the total weight percent amount of all components, substances or agents of the antimicrobial skin care liquid composition as well as use composition are selected such that it does not exceed 100 wt.-%.

It is understood that, as used here, "percent", "%", and the like are intended to be synonymous with "weight percent", "wt-%", etc..

As used herein, the term "about" refers to variation in the numerical quantity that can occur, for example, through typical measuring and liquid handling procedures used for making the antimicrobial skin care liquid composition in the real world; through inadvertent error in these procedures; through differences in the manufacture, source, or purity of the ingredients used to make the compositions or carry out the methods; and the like. The term "about" also encompasses amounts that differ due to different equilibrium conditions for a antimicrobial skin care liquid composition resulting from a particular initial mixture.

Whether or not, modified by the term "about", the claims include equivalents to the quantities.

It should be noted that, as used in this specification and the appended claims, the singular forms "a", "an", and "the" include plural referents unless the content clearly dictates otherwise.

It should also be noted that the term "or" is generally employed in its sense including "and/or" unless the content clearly dictates otherwise.

### Compositions Used in a Teat Dip Embodiment of the Invention

The compositions of the invention can be formulated as a teat dip for mastitis prevention or control. According to this aspect, the antimicrobial skin care liquid composition, useful in cold temperature environments, comprises:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C₂ to C₆ organic monoalcohol, selected from the group comprising ethanol, n-propanol, isopropanol, n-butanol, sec-butanol, iso-butanol, tert-butanol, pentanol and/or hexanol;
- ≥ 0.1 wt.-% to ≤ 10 wt.-% of sorbitol; and
- ≥ 0.1 wt.-% to ≤ 15 wt.-% of at least one C2 to C6 organic acid; wherein
the weight-% of the components are based on the total weight amount of the antimicrobial skin care liquid composition.

According to another aspect of the present invention the antimicrobial skin care liquid composition, useful in cold temperature environments, comprises:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C2 to C6 organic monoalcohol selected from the group comprising ethanol, n-propanol, isopropanol, n-butanol, sec-butanol, iso-butanol, tert-butanol, pentanol and/or hexanol;
- ≥ 0.1 wt.-% to ≤ 10 wt.-% of sorbitol;
- ≥ 0.1 wt.-% to ≤ 15 wt.-% of at least one C2 to C6 organic acid, preferably lactic acid;
- ≥ 0 wt.-% water and preferably the rest is water; wherein
the weight-% of the components are based on the total weight amount of the antimicrobial skin care liquid composition.

According to another aspect of the present invention the antimicrobial skin care liquid composition, useful in cold temperature environments, comprises:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C2 to C6 organic monoalcohol selected from the group comprising ethanol, n-propanol, isopropanol, n-butanol, sec-butanol, iso-butanol, tert-butanol, pentanol and/or hexanol;
- ≥ 0.1 wt.-% to ≤ 10 wt.-% of sorbitol;
- ≥ 0.1 wt.-% to ≤ 15 wt.-%, in particular ≥ 0.4 wt.-% to ≤ 13 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one C2 to C6 organic acid, preferably of at least one C₂ to Cg and further preferred of at least one C₄ to C₆ organic acid, whereby lactic acid is most preferred;
- ≥ 0 wt.-% water and preferably the rest is water; wherein
the weight-% of the components are based on the total weight amount of the antimicrobial skin care liquid composition.

According to another aspect of the present invention the antimicrobial skin care liquid composition, useful in cold temperature environments, comprises:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C2 to C6 organic monoalcohol selected from the group comprising ethanol, n-propanol, isopropanol, n-butanol, sec-butanol, iso-butanol, tert-butanol, pentanol and/or hexanol;
- ≥ 0.1 wt.-% to ≤ 10 wt.-% of sorbitol;
- ≥ 0.1 wt.-% to ≤ 15 wt.-%, in particular ≥ 0.4 wt.-% to ≤ 13 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one C2 to C6 organic acid, preferably of at least one C₂ to C₈ and further preferred of at least one C₄ to C₆ organic acid, whereby lactic acid is most preferred;
- ≥ 0 wt.-% to ≤ 5 wt.-%, preferably ≥ 0.1 wt.-% to ≤ 3 wt.-%, further preferred ≥ 0.5 wt.-% to ≤ 2 wt.-%, and more preferred ≥ 1 wt.-% to ≤ 1.5 wt.-%, of at least one surfactant, preferably an anionic surfactant, more preferred an C₆ to C₁₈ ether sulfate, and most preferred an lauryl ether sulfate with 2 EO;
- ≥ 0 wt.-% to ≤ 30 wt.-%, preferably ≥ 0.01 wt.-% to ≤ 20 wt.-%, further preferred ≥ 1 wt.-% to ≤ 10 wt.-%, in addition preferred ≥ 3 wt.-% to ≤ 8 wt.-%, and more preferred ≥ 5 wt.-% to ≤ 7 wt.-%, of at least one chlorite generating compound, preferably an alkali and/or alkaline earth hypochlorite, and more preferred NaClO₂; and
- ≥ 0 wt.-% water and preferably the rest is water; wherein
the weight-% of the components are based on the total weight amount of the antimicrobial skin care liquid composition.

According to another aspect of the present invention the antimicrobial skin care liquid composition, useful in cold temperature environments, comprises:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C2 to C6 organic monoalcohol selected from the group comprising ethanol, n-propanol, isopropanol, n-butanol, sec-butanol, iso-butanol, tert-butanol, pentanol and/or hexanol;
- ≥ 0.1 wt.-% to ≤ 10 wt.-% of sorbitol;
- ≥ 0.1 wt.-% to ≤ 15 wt.-%, in particular ≥ 0.4 wt.-% to ≤ 13 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one C2 to C6 organic acid, preferably of at least one C₂ to C₈ and further preferred of at least one C₄ to C₆ organic acid, whereby lactic acid is most preferred;
- ≥ 0 wt.-% to ≤ 30 wt.-%, preferably ≥ 0.01 wt.-% to ≤ 20 wt.-%, further preferred ≥ 1 wt.-% to ≤ 10 wt.-%, in addition preferred ≥ 3 wt.-% to ≤ 8 wt.-%, and more preferred ≥ 5 wt.-% to ≤ 7 wt.-%, of at least one chlorite generating compound, preferably an alkali and/or alkaline earth hypochlorite, and more preferred NaClO₂;
- ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one alkali source, preferably triethanolamine; and
- ≥ 0 wt.-% water and preferably the rest is water; wherein
the weight-% of the components are based on the total weight amount of the antimicrobial skin care liquid composition.

According to another aspect of the present invention the antimicrobial skin care liquid composition, useful in cold temperature environments, comprises:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C2 to C6 organic monoalcohol selected from the group comprising ethanol, n-propanol, isopropanol, n-butanol, sec-butanol, iso-butanol, tert-butanol, pentanol and/or hexanol;
- ≥ 0.1 wt.-% to ≤ 10 wt.-% of sorbitol;
- ≥ 0.1 wt.-% to ≤ 15 wt.-%, in particular ≥ 0.4 wt.-% to ≤ 13 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one C2 to C6 organic acid, preferably of at least one C₂ to Cg and further preferred of at least one C₄ to C₆ organic acid, whereby lactic acid is most preferred;
- ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one hydrotrope, preferably an aromatic hydrocarbon sulfonate, xylene sulfonate, toluene sulfonate, cumene sulfonate, and/or n-octane sulfonate and most preferred cumene sulfonate;
- ≥ 0 wt.-% to ≤ 30 wt.-%, preferably ≥ 0.01 wt.-% to ≤ 20 wt.-%, further preferred ≥ 1 wt.-% to ≤ 10 wt.-%, in addition preferred ≥ 3 wt.-% to ≤ 8 wt.-%, and more preferred ≥ 5 wt.-% to ≤ 7 wt.-%, of at least one chlorite generating compound, preferably an alkali and/or alkaline earth hypochlorite, and more preferred NaClO₂; and
- ≥ 0 wt.-% water and preferably the rest is water; wherein
the weight-% of the components are based on the total weight amount of the antimicrobial skin care liquid composition.

According to another aspect of the invention the antimicrobial skin care liquid composition comprises at least ≥ 15 wt.-% to ≤ 100 wt.-%, preferably ≥ 20 wt.-% to ≤ 90 wt.-%, further preferred ≥ 25 wt.-% to ≤ 80 wt.-%, also preferred ≥ 30 wt.-% to ≤ 70 wt.-%, in addition preferred ≥ 40 wt.-% to ≤ 70 wt.-%, and finally preferred ≥ 50 wt.-% to ≤ 60 wt.-%, of a mixture comprising:
- glycerin,
- of propylene glycol,
- of at least one C2 to C6 organic monoalcohol selected from the group comprising ethanol, n-propanol, isopropanol, n-butanol, sec-butanol, iso-butanol, tert-butanol, pentanol and/or hexanol, and
- of sorbitol.

According to another aspect of the invention the antimicrobial skin care liquid composition comprises at least ≥ 15 wt.-% to ≤ 100 wt.-%, preferably ≥ 20 wt.-% to ≤ 90 wt.-%, further preferred ≥ 25 wt.-% to ≤ 80 wt.-%, also preferred ≥ 30 wt.-% to ≤ 70 wt.-%, in addition preferred ≥ 40 wt.-% to ≤ 70 wt.-%, and finally preferred ≥ 50 wt.-% to ≤ 60 wt.-%, of a mixture comprising:
- glycerin;
- propylene glycol;
- at least one C₂ to C₆ organic monoalcohol selected from the group comprising ethanol, n-propanol, isopropanol, n-butanol, sec-butanol, iso-butanol, tert-butanol, pentanol and/or hexanol, preferably n-propanol and/or isopropanol, wherein isopropanol is most preferred; and
- sorbitol.

According to another aspect of the invention the antimicrobial skin care liquid composition comprises:
- ≥ 5 wt.-% to ≤ 25 wt.-%, preferably ≥ 10 wt.-% to ≤ 20 wt.-%, and more preferred ≥ 13 wt.-% to ≤ 16 wt.-%, of glycerin;
- ≥ 0.1 wt.-% to ≤ 20 wt.-%, preferably ≥ 2 wt.-% to ≤ 10 wt.-%, and more preferred ≥ 3 wt.-% to ≤ 6 wt.-%, of propyleneglycol;
- ≥ 0.1 wt.-% to ≤ 20 wt.-%, preferably ≥ 2 wt.-% to ≤ 10 wt.-%, and more preferred ≥ 3 wt.-% to ≤ 6 wt.-%, of preferably propanol and more preferred isopropanol;
- ≥ 0.1 wt.-% to ≤ 10 wt.-%, preferably ≥ 0.5 wt.-% to ≤ 5.0 wt.-%, and more preferred ≥ 1 wt.-% to ≤ 2.5 wt.-%, of sorbitol;
- ≥ 0 wt.-% to ≤ 5 wt.-%, preferably ≥ 0.1 wt.-% to ≤ 3 wt.-%, further preferred ≥ 0.5 wt.-% to ≤ 2 wt.-%, and more preferred ≥ 1 wt.-% to ≤ 1.5 wt.-%, of at least one anionic surfactant, more preferred an C₆ to C₁₈ ether sulfate, and most preferred an laurylethersulfate with 2 EO;
- ≥ 0.1 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one C2 to C6 organic acid, preferably lactic acid;
- ≥ 0 wt.-% to ≤ 30 wt.-%, preferably ≥ 0.01 wt.-% to ≤ 20 wt.-%, further preferred ≥ 1 wt.-% to ≤ 10 wt.-%, in addition preferred ≥ 3 wt.-% to ≤ 8 wt.-%, and more preferred ≥ 5 wt.-% to ≤ 7 wt.-%, of at least one chlorite generating compound, preferably an alkali and/or alkaline earth hypochlorite, and more preferred NaClO₂;
- ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one alkali source, preferably triethanolamine;
- ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one hydrotrope, preferably an aromatic hydrocarbon sulfonate, xylene sulfonate, toluene sulfonate, cumene sulfonate, and/or n-octane sulfonate and most preferred cumene sulfonate; and
- ≥ 0 wt.-% water; wherein
the weight-% of the components are based on the total weight amount of the antimicrobial skin care liquid composition.

### Components Used in a Teat Dip Embodiment of the Invention

The preferred compositions of the invention comprise ingredients, which are generally regarded as safe, and are not of themselves or in admixture incompatible with milk or milk by-products. Ingredients may also be selected which are cooperative in their combined effects whether incorporated for physical integrity of the formulation or to facilitate healing and overall health of the teat.

The antimicrobial skin care liquid composition of the invention can be free of surfactant/s, such as nonionic tensides, anionic tensides, cationic tensides and amphoteric tensides.

It should be understood that the antimicrobial skin care liquid composition of the invention can be free of a hydrotrope component.

It should be understood that the antimicrobial skin care liquid composition of the invention can be free of a phosphate.

It should be understood that the antimicrobial skin care liquid composition of the invention can be free of at least one additive, preferably all additives, selected from the group fragrances, dyes, antistatic agents, UV absorbers, reducing agents and/or buffering compounds.

### C₃ to C₆ organic triolalcohol

The C₃ to C₆ organic triolalcohol is glycerin.

The C₃ to C₆ organic triolalcohol lowers the freezing point of the antimicrobial skin care liquid compositions of the invention and are useful to facilitate faster repair of teats that are already in poor health conditions, at deep temperatures up to - 15° C or preferably up to - 20° C, if used in a mixture of the monoalcohol, diolalcohol and sugar alcohol of the composition of the invention due to their surprising synergetic effect.

In general, the glycerin, of the antimicrobial skin care liquid composition of the invention can be present at ≥ 5 wt.-% to ≤ 25 wt.-%, preferably ≥ 10 wt.-% to ≤ 20 wt.-%, and more preferred ≥ 13 wt.-% to ≤ 16 wt.-%; based on the total weight amount of the antimicrobial skin care liquid composition.

### C₂ to C₆ organic diolalcohol

The C₂ to C₆ organic diolalcohol is propyleneglycol.

The C₂ to C₆ organic diolalcohol lowers the freezing point of the antimicrobial skin care liquid compositions of the invention and are useful to facilitate faster repair of teats that are already in poor health conditions, at deep temperatures up to - 15° C or preferably up to - 20° C, if used in a mixture of the monoalcohol, trialcohol and sugar alcohol of the composition of the invention due to their surprising synergetic effect.

In general, the propyleneglycol, of the antimicrobial skin care liquid composition of the invention can be present at ≥ 0.1 wt.-% to ≤ 20 wt.-%, preferably ≥ 2 wt.-% to ≤ 10 wt.-%, and more preferred ≥ 3 wt.-% to ≤ 6 wt.-%, based on the total weight of the antimicrobial skin care liquid composition of the invention.

### C₂ to C₆ organic monoalcohol

The at least one C₂ to C₆ organic monoalcohol can be selected from the group comprising ethanol, n-propanol, isopropanol, n-butanol, sec-butanol, iso-butanol, tert-butanol, pentanol and/or hexanol. Preferably, the at least one C₂ to C₆ organic monoalcohol can be n-propanol and/or isopropanol, wherein isopropanol is most preferred.

The C₂ to C₆ organic monoalcohol has an antiseptic effect and lowers the freezing point of the antimicrobial skin care liquid compositions of the invention, useful to disinfect teats at deep temperatures up to - 15° C or preferably up to for example - 20° C, if used in a mixture of the diolalcohol, trialcohol and sugar alcohol of the composition of the invention due to their surprising synergetic effect.

In general, the at least one C2 to C6 organic monoalcohol selected from the group comprising ethanol, n-propanol, isopropanol, n-butanol, sec-butanol, iso-butanol, tert-butanol, pentanol and/or hexanol, preferably n-propanol and/or isopropanol, wherein isopropanol is most preferred, of the antimicrobial skin care liquid composition of the invention can be present at ≥ 0.1 wt.-% to ≤ 20 wt.-%, preferably ≥ 2 wt.-% to ≤ 10 wt.-%, and more preferred ≥ 3 wt.-% to ≤ 6 wt.-%, based on the total weight of the antimicrobial skin care liquid composition of the invention.

### Sugar Alcohol

The at least one sugar alcohol can have the formula I:

HOCH₂[CH(OH)]ₙCH₂OH;

wherein n = 2 to 6, in particular n = 3 to 5, or preferably n = 4. However, most preferred the sugar alcohol is sorbitol.

Teat dip compositions of the present invention can also include a sugar alcohol to moisturize and generally reduce irritation and promote the healing of the teat surface of which may result either from the antimicrobial component, from the mechanical action of the milking machine or from environmental conditions such as frigid temperatures wind chill, dehydration, abrasion, windburn and sunburn.

The sugar alcohol of formula I has a moisturizing effect and lowers the freezing point of the antimicrobial skin care liquid compositions of the invention, useful to facilitate faster repair of teats that are already in poor health conditions, at deep temperatures up to - 15° C or preferably up to - 20° C, if used in a mixture of the monoalcohol, diolalcohol, and trialcohol of the composition of the invention due to their surprising synergetic effect.

In general, the at least one sugar alcohol of formula I, preferably sorbitol, of the antimicrobial skin care liquid composition of the invention can be present at ≥ 0.1 wt.-% to ≤ 10 wt.-%, preferably ≥ 0.5 wt.-% to ≤ 5.0 wt.-%, and more preferred ≥ 1 wt.-% to ≤ 2.5 wt.-%, based on the total weight of the antimicrobial skin care liquid composition of the invention.

### Water

The antimicrobial skin care liquid composition comprises in addition water. In general, the antimicrobial skin care liquid composition of the invention can comprise water at ≥ 0 wt.-%. Preferably the component water is added as a rest. The term as "a rest" means that component water is added to the other components in amount such that all components of the antimicrobial skin care liquid composition of the invention are in total 100 wt.-%, based on the total weight of the antimicrobial skin care liquid composition of the invention.

### Surfactant

The antimicrobial skin care liquid composition may include anionic surfactants, nonionic surfactants, kationic surfactants, amphoteric surfactants and mixtures thereof.

As used herein, "surfactant" refers to any agent that lowers the surface tension of a liquid, for example water. Exemplary surfactants which may be suitable for use with the present invention are described below. In one embodiment, surfactants may be selected from the group consisting of anionic, nonionic, cationic, amphoteric, zwitterionic, and combinations thereof. In other non limiting embodiments, the surfactant may be an anionic surfactant.

The surfactant component can be used to reduce surface tension and wet the teat surface.

### Nonionic Surfactants

Exemplary nonionic surfactants that can be used in the antimicrobial skin care liquid composition of the invention may be alkoxylated, preferably ethoxylated or ethoxylated and propoxylated, fatty acid alkyl esters preferably containing 1 to 4 carbon atoms in the alkyl chain, more particularly the fatty acid methyl esters.

Further surfactants include ethoxylated long chain fatty acid amides where the fatty acid has 8-20 carbon atoms and the amide group is ethoxylated with 1-20 ethylene oxide units.

A further class of nonionic surfactants, which can be used according to the invention, is that of the alkyl polyglycosides (APG). Suitable alkyl polyglycosides satisfy the general Formula RO(G)z where R is a linear or branched, particularly 2-methyl-branched, saturated or unsaturated aliphatic radical containing 8 to 22 and preferably 12 to 18 carbon atoms and G stands for a glycose unit containing 5 or 6 carbon atoms, preferably glucose. The degree of oligomerization z is a number between 1.0 and 4.0 and preferably between 1.1 and 1.4.

Additionally, non-ionic surfactants derived from the condensation of ethylene oxide with the product resulting from the reaction of propylene oxide and ethylene diamine are also useful. For example, there are compounds containing from 40% to 80% of polyoxyethylene by weight and having a molecular weight from 5,000 to 11,000 resulting from the reaction of ethylene oxide groups with a hydrophobic base constituted of the reaction product from ethylene diamine and excess propylene oxide wherein the base has a molecular weight on order of 2,500-3, 000.

Suitable nonionic surfactants include the polyoxyethylene-polyoxypropylene condensates, which are sold by BASF under the trade name'Pluronic', polyoxyethylene condensates of aliphatic alcohols/ethylene oxide condensates having from 1 to 30 moles of ethylene oxide per mole of coconut alcohol; ethoxylated long chain alcohols sold by Shell Chemical Co. under the trade name 'Neodol', polyoxyethylene condensates of sorbitan fatty acids, alkanolamides, such as the monoalkoanolamides, dialkanolamides and the ethoxylated alkanolamides, for example coconut monoethanolamide, lauric isopropanolamide and lauric diethanolamide; and amine oxides for example dodecyldimethylamine oxide.

Nonionic surfactants that can be used in the composition of the invention include polyalkylene oxide surfactants (also known as polyoxyalkylene surfactants or polyalkylene glycol surfactants). Suitable polyalkylene oxide surfactants include polyoxypropylene surfactants and polyoxyethylene glycol surfactants. Suitable surfactants of this type are synthetic organic polyoxypropylene (PO)-polyoxyethylene (EO) block copolymers. These surfactants include a di-block polymer comprising an EO block and a PO block, a center block of polyoxypropylene units (PO), and having blocks of polyoxyethylene grafted onto the polyoxypropylene unit or a center block of EO with attached PO blocks. Further, this surfactant can have further blocks of either polyoxyethylene or polyoxypropylene in the molecules. A suitable average molecular weight range of useful surfactants can be 1,000 to 40,000 and the weight percent content of ethylene oxide can be 10-80 wt %.

A suitable polyethylene glycol for use in the present invention can have an average mol weight (MW) in the range of ≥ 4000 to ≤ 12000, preferably ≥ 6000 to ≤ 10000 and more preferred of ≥ 7000 to ≤ 8000. Polyethylene glycol that can be used are marketed for example by BASF under the tradename PLURIOL®.

According to the invention, the antimicrobial skin care liquid composition may comprises at least one polyethylene glycol, preferably a polyethylene glycol with an average mol weight in the range of 4.000 to 12.000, and more preferred a polyethylene glycol having an average mol weight of 8,000.

Further exemplary non-ionic surfactants include alkylphenol alkoxylates, and amine oxides such as alkyl dimethylamine oxide or bis(2- hydroxyethyl) alkylamine oxide.

The nonionic surfactants can be provided in the composition in an amount of ≥ 0 wt.-% to ≤ 5 wt.-%, preferably ≥ 0.1 wt.-% to ≤ 3 wt.-%, further preferred ≥ 0.5 wt.-% to ≤ 2 wt.-%, and more preferred ≥ 1 wt.-% to ≤ 1.5 wt.-%, based on the weight of the total antimicrobial skin care liquid composition.

It should be understood that the antimicrobial skin care liquid composition of the invention can be preferably free of a non-ionic surfactant.

### Anionic surfactant

The antimicrobial skin care liquid composition can contain an additional anionic surfactant component that includes a detersive amount of an anionic surfactant or a mixture of anionic surfactants. Anionic surfactants are desirable in antimicrobial skin care liquid compositions because of their wetting and detersive properties. The anionic surfactants that can be used according to the invention include any anionic surfactant available in the skin care field. Suitable groups of anionic surfactants include sulfonates and sulfates. Suitable surfactants that can be provided in the anionic surfactant component include alkyl aryl sulfonates, secondary alkane sulfonates, alkyl methyl ester sulfonates, alpha olefin sulfonates, alkyl ether sulfates, alkyl sulfates, and alcohol sulfates.

Suitable alkyl aryl sulfonates that can be used in the antimicrobial skin care liquid composition can have an alkyl group that contains 6 to 24 carbon atoms and the aryl group can be at least one of benzene, toluene, and xylene. A suitable alkyl aryl sulfonate includes linear alkyl benzene sulfonate. A suitable linear alkyl benzene sulfonate includes linear dodecyl benzyl sulfonate that can be provided as an acid that is neutralized to form the sulfonate. Additional suitable alkyl aryl sulfonates include xylene sulfonate and cumene sulfonate. Suitable alkane sulfonates that can be used in the antimicrobial skin care liquid composition can have an alkane group having 6 to 24 carbon atoms. Suitable alkane sulfonates that can be used include secondary alkane sulfonates. A suitable secondary alkane sulfonate includes sodium C14-C17 secondary alkyl sulfonate commercially available as Hostapur SAS from Clariant. Suitable alkyl methyl ester sulfonates that can be used in the antimicrobial skin care liquid composition include those having an alkyl group containing 6 to 24 carbon atoms. Suitable alpha olefin sulfonates that can be used in the antimicrobial skin care liquid composition include those having alpha olefin groups containing 6 to 24 carbon atoms.

Suitable alkyl ether sulfates that can be used in the antimicrobial skin care liquid composition include those having between 1 and 10 repeating alkoxy groups, between 1 and 5 repeating alkoxy groups. In general, the alkoxy group will contain between 2 and 4 carbon atoms. A suitable alkoxy group is ethoxy. A suitable alkyl ether sulfate is sodium lauryl ether sulfate and is available under the name Steol CS-460.

Suitable alkyl sulfates that can be used in the antimicrobial skin care liquid composition include those having an alkyl group containing 6 to 24 carbon atoms. Suitable alkyl sulfates include, but are not limited to, sodium lauryl sulfate and sodium lauryl/myristyl sulfate.

Suitable alcohol sulfates that can be used in the antimicrobial skin care liquid composition include those having an alcohol group containing 6 to 24 carbon atoms.

The anionic surfactant can be neutralized with an alkaline metal salt, an amine, or a mixture thereof. Suitable alkaline metal salts include sodium, potassium, and magnesium. Suitable amines include monoethanolamine, triethanolamine, and monoisopropanolamine. If a mixture of salts is used, a suitable mixture of alkaline metal salt can be sodium and magnesium, and the molar ratio of sodium to magnesium can be between 3:1 and 1:1.

The antimicrobial skin care liquid composition can include the anionic surfactant component in an amount sufficient to provide a composition having desired wetting and detersive properties. The antimicrobial skin care liquid composition contain ≥ 0 wt.-% to ≤ 5 wt.-%, preferably ≥ 0.1 wt.-% to ≤ 3 wt.-%, further preferred ≥ 0.5 wt.-% to ≤ 2 wt.-%, and more preferred ≥ 1 wt.-% to ≤ 1.5 wt.-%, of at least one anionic surfactant, more preferred an C₆ to C₁₈ ether sulfate, and most preferred an lauryl ether sulfate with 2 EO

It should be understood that the antimicrobial skin care liquid composition of the invention can be preferably free of an anionic surfactant.

### Cationic Surfactans

The antimicrobial skin care liquid composition can contain a cationic surfactant component that includes a detersive amount of cationic surfactant or a mixture of cationic surfactants. The cationic surfactant can be used to provide sanitizing properties. Cationic surfactants that can be used in the antimicrobial skin care liquid composition include, but are not limited to: amines such as primary, secondary and tertiary monoamines with C1-8 alkyl or alkenyl chains, ethoxylated alkylamines, alkoxylates of ethylenediamine, imidazoles such as a 1-(2-hydroxyethyl)-2-imidazoline, a 2-alkyl-1-(2-hydroxyethyl)-2-imidazoline, and the like; and poly sulfonate ammonium salts, as for example, alkylpoly sulfonate ammonium chloride surfactants such as n-alkyl(C12-C18)dimethylbenzyl ammonium chloride, n-tetradecyldimethylbenzylammonium chloride monohydrate, and a naphthylene-substituted poly sulfonate ammonium chloride such as dimethyl-1-naphthylmethylammonium chloride. Suitable cationic surfactants include quaternary ammonium compounds having the formula of RR'R"R'''N⁺X⁻, where R, R', R" and R''' are each a C₁-C₂₄ alkyl, aryl or arylalkyl group that can optionally contain one or more P, O, S or N heteroatoms, and X is F, Cl, Br, I or an alkyl sulfate.

Additional preferred cationic surfactants include ethoxylated and/or propoxylated alkyl amines, diamines, or triamines.

Each of R, R', R" and R''' can independently include, individually or in combination, substituents including 6 to 24 carbon atoms, preferably 14 to 24 carbon atoms, and more preferably, 16 to 24 carbon atoms.

Each of R, R', R" and R''' can independently be linear, cyclic, branched, saturated, or unsaturated, and can include heteroatoms such as oxygen, phosphorous, sulfur, or nitrogen. Any two of R, R', R" and R''' can form a cyclic group. Any one of three of R, R', R" and R''' can independently be hydrogen. X is preferably a counter ion and preferably a non-fluoride counter ion. Exemplary counter ions include chloride, bromide, methosulfate, ethosulfate, sulfate, and phosphate.

In an embodiment, the quaternary ammonium compound includes alkyl ethoxylated and/or propoxylated quaternary ammonium salts (or amines).

Preferably, the alkyl group contains between 6 and 22 carbon atoms and can be saturated and/or unsaturated. The degree of ethoxylation is preferably between 2 and 20, and/or the degree of propoxylation is preferably between 0 and 30.
In an embodiment, the quaternary ammonium compound includes an alkyl group with 6 to 22 carbon atoms and a degree of ethoxylation between 2 and 20. A preferred cationic surfactant is commercially available under the name Berol 563 from Akzo-Nobel.

The cationic surfactants can be provided in the composition in an amount of ≥ 0 wt.-% to ≤ 5 wt.-%, preferably ≥ 0.1 wt.-% to ≤ 3 wt.-%, further preferred ≥ 0.5 wt.-% to ≤ 2 wt.-%, and more preferred ≥ 1 wt.-% to ≤ 1.5 wt.-%, based on the weight of the total antimicrobial skin care liquid composition.

It should be understood that the antimicrobial skin care liquid composition of the invention can be preferably free of a cationic surfactant.

### Amphoteric Surfactants

Amphoteric surfactants can also be used to provide desired detersive properties. Suitable amphoteric surfactants that can be used include, but are not limited to: betaines, imidazolines, and propionates. Suitable amphoteric surfactants include, but are not limited to: sultaines, amphopropionates, amphodipropionates, aminopropionates, aminodipropionates, amphoacetates, amphodiacetates, and amphohydroxypropylsulfonates. When the detergent composition includes an amphoteric surfactant, the amphoteric surfactant can be included in an amount of ≥ 0 wt.-% to ≤ 5 wt.-%, preferably ≥ 0.1 wt.-% to ≤ 3 wt.-%, further preferred ≥ 0.5 wt.-% to ≤ 2 wt.-%, and more preferred ≥ 1 wt.-% to ≤ 1.5 wt.-%, based on the weight of the total antimicrobial skin care liquid composition.

It should be understood that the antimicrobial skin care liquid composition of the invention can be preferably free of an amphoteric surfactant.

### Hydrotrophe

Solubilizing intermediaries called hydrotropes may be present in the antimicrobial skin care liquid composition of the invention. Hydrotropes that can be suitable used are selected from the group comprising aromatic hydrocarbon sulfonate, preferably xylene sulfonate, toluene sulfonate, or cumene sulfonate; or n-octane sulfonate; or their sodium-, potassium- or ammonium salts or as salts of organic ammonium bases.

In some preferred embodiments the hydrotrope may be selected from the group comprising of a xylene sulfonate, toluene sulfonate, or cumene sulfonate, n-octane sulfonate, and/or acids thereof and also preferred cumene sulfonate.

In some embodiments, Na-cumolsulfonat, linear alkylbenzene sulfonates (LAS) and/or xylene sulfonate, cumolsulfonate may be suitable to use as hydrotrope and having an improved solubilizing effect.

In some embodiments, the antimicrobial skin care liquid composition comprises of ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one hydrotrope, preferably an aromatic hydrocarbon sulfonate, xylene sulfonate, toluene sulfonate, cumene sulfonate, and/or n-octane sulfonate and most preferred cumene sulfonate, by weight of the total antimicrobial skin care liquid composition.

It should be understood that the hydrotrope can present in the form of an acid or salt thereof, depending on the pH of the antimicrobial skin care liquid composition.

It should be understood that the antimicrobial skin care liquid composition of the invention can be preferably free of a hydrotrope.

### Alkaline Source

The source of alkalinity can be any source of alkalinity that is compatible with the other components of the antimicrobial skin care liquid composition and that will provide the solution with the desired pH.

Exemplary sources of alkalinity include alkali metal hydroxides, alkali metal salts, phosphates, amines, and mixtures thereof.

Exemplary alkali metal hydroxides include sodium hydroxide, potassium hydroxide, and lithium hydroxide.

Exemplary alkali metal salts include sodium carbonate, trisodium phosphate, potassium carbonate, and mixtures thereof.

Exemplary phosphates include sodium pyrophosphate, potassium pyrophosphate, and mixtures thereof.

Exemplary amines include alkanolamine selected from the group comprising triethanolamine, monoethanolamine, diethanolamine, and mixtures thereof.

The source of alkalinity, preferably an alkali metal hydroxide, may be added to the antimicrobial skin care liquid composition in a variety of forms, including for example in the form of solid beads, dissolved in an aqueous solution or a combination thereof. Alkali metal hydroxides are commercially available as pellets or beads having a mix of particle sizes ranging from 12-100 U. S. mesh, or as an aqueous solution, as for example, as 45 wt. %, 50 wt. % and 73 wt. % solution.

According to the invention, the antimicrobial skin care liquid composition may comprises ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one alkali source, preferably triethanolamine; by weight of the total antimicrobial skin care liquid composition.

It should be understood that the antimicrobial skin care liquid composition of the invention can be preferably free of an alkali source.

### C2 to C6 organic acid

C2 to C6 organic acids having a biocidal activity may be present in the antimicrobial skin care liquid composition of the invention. C2 to C6 organic acids, further preferred C₄ to C₆ organic acid, that can be suitable used are selected from the group comprising formic acid, acetic acid, propionic acid, iso-propionic acid, butanoic acid, pentanoic acid, hexanoic acid, heptanoic acid, octanoic acid, nonanoic acid, decanoic acid, glycolic acid, citric acid, lactic acid, tartaric acid, fumaric acid, malic acid, itaconic acid, ascorbic acid, benzoic acid, salicylic acid, and/or succinic acid.

More preferred the organic acid that can be used is formic acid, lactic acid and/or benzoic acid.

According to the invention, the antimicrobial skin care liquid composition may comprises of ≥ 0.1 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one C2 to C6 organic acid, preferably of at least one C₂ to Cg and further preferred of at least one C₄ to C₆ organic acid, whereby lactic acid is most preferred.

### Chlorite Generating Compound

The antimicrobial skin care liquid composition may comprises at least one chlorite generating compound. The chlorite generating compound can be selected from the group of alkali and alkaline earth hypochlorites, and most preferred the chlorite generating compound is NaClO₂.

According to the invention, the antimicrobial skin care liquid composition may comprises of ≥ 0 wt.-% to ≤ 30 wt.-%, preferably ≥ 0.01 wt.-% to ≤ 20 wt.-%, further preferred ≥ 1 wt.-% to ≤ 10 wt.-%, in addition preferred ≥ 3 wt.-% to ≤ 8 wt.-%, and more preferred ≥ 5 wt.-% to ≤ 7 wt.-%, of at least one chlorite generating compound, preferably an alkali and/or alkaline earth hypochlorite, and more preferred NaClO₂.

### Further Antimicrobial Agents

Numerous inorganic and organic antimicrobial agents may be utilized in teat dip compositions including but not limited to bromine release compounds, e.g. alkali and alkaline earth hypobromites, isocyanurates, brominated derivatives of hydantoin, sulfamide, amine, etc., quaternary ammonium compounds, chlorhexidine salts, peroxide and peroxyacid compounds and acidified anionic surfactants. Of these typically applied antimicrobial agents that have been investigated for control of bovine mastitis, acidified alkylaryl sulfonates are proven efficacious against mastitis causing microorganisms; and, are preferred as additional compound in compositions of the present invention. More specifically, dodecylbenzene sulfonic acid is especially preferred as additional antimicrobial agents.

According to the invention, the antimicrobial skin care liquid composition may comprises of ≥ 0 wt.-% to ≤ 30 wt.-%, preferably ≥ 0.01 wt.-% to ≤ 20 wt.-%, further preferred ≥ 1 wt.-% to ≤ 10 wt.-%, in addition preferred ≥ 3 wt.-% to ≤ 8 wt.-%, and more preferred ≥ 5 wt.-% to ≤ 7 wt.-%, of at least one Antimicrobial Agent.

### pH

The preferred pH for a teat dip antimicrobial skin care liquid composition of the invention is ≥ 2 pH to ≤ 6 pH, preferably ≥ 3.0 pH to ≤ 5.5 pH, further preferred ≥ 3.5 pH to ≤ 5 pH, and more preferred ≥ 4 pH to ≤ 4.5 pH. If the pH is less than the preferred range, a base such as triethanol amine can be added incrementally until the appropriate pH is achieved. If the pH is greater than the preferred range, an acid, such as lactic acid, can be added incrementally until the appropriate pH is obtained.

The following examples are provided to further describe certain advantageous compositions according to the invention.

### Examples

### Example 1 Formulation of Teat Dip Composition

The present Example provides one procedure for preparing a working composition of a teat dip composition according to one embodiment of the invention. This procedure can be used regardless of the total weight of the composition formulated. Thus, while a particular weight percentage of a component may vary among formulations, the procedure used for mixing the components is the same. It will be appreciated that other procedures can be used and are within the knowledge of one skilled in the art.

Deionized water is added to a stainless steel tank having a variable speed pitched blade turbine. The tank is agitated and the components sorbitol in an aqueous solution, glycerin, propylene glycol and isopropanol are added with 15 minutes of mixing

Tables 1-3 provide exemplarily formulations for some teat dip compositions according to the invention E1 to E13 and comparison formulations C1 to C5.

**Table 1: Teat Dip Formulations C1 to C5 of organic acid free compositions**

| Wt.-% | C1 | C2 | C3 | C4 | C5 |
|---|---|---|---|---|---|
| Dest. Water | 79.9 | 83.4 | 76.4 | 76.4 | 94.7 |
| Sorbitol | 0.1 | 1.6 | 1.6 | 1.6 | 0.1 |
| **Glycerin** | 15 | 5 | 15 | 15 | 5 |
| Propylene glycol | 5 | 5 | 2 | 5 | 0.1 |
| Isopropanol | 5 | 5 | 5 | 0.1 | 0.1 |
| freezing at -8° C | * | * | * | * | * |
| visc. at -20° C /mPas (Brookfield; spdl. 61; 12 rpm) | | | | | |
| visc. at -8° C /mPas (Brookfield; spdl. 61; 12 rpm) | | | | | |

| | | | | | |
|---|---|---|---|---|---|
| * = frozen | | | | | |

Table 1 shows that organic acid free compositions are frozen at -8° C and cannot be used as teat dip formulations according to the invention.

**Table 2: Teat Dip Formulations E1 to E6 of the invention**

| Wt.-% | **E1** | **E2** | **E3** | **E4** | **E5** | **E6** | **E7** |
|---|---|---|---|---|---|---|---|
| Dest. Water | 73.9 | 78.6 | 75.4 | 57.4 | 57.4 | 62.4 | 62.4 |
| Sorbitol | 0.1 | 0.4 | 1.6 | 1.6 | 1.6 | 1.6 | 2 |
| Glycerin | 15 | 15 | 15 | 15 | 15 | 25 | 15 |
| Propylene glycol | 5 | 5 | 2 | 20 | 5 | 5 | 5 |
| Isopropanol | 5 | 5 | 5 | 5 | 20 | 5 | 5 |
| Lactic acid | 1 | 1 | 1 | 1 | 1 | 1 | 8 |
| freezing at -15° C | +* | +* | - | + | + | + | + |
| freezing at -12° C | + | + | + | + | + | + | + |
| freezing at-10° C | + | + | + | + | + | + | + |
| freezing at -8° C | + | + | + | + | + | + | + |
| visc. at -12° C /mPas (Brookfield; spdl. 61; 12 rpm) | B | B | C | A | A | A | A |

| | | | | | | | |
|---|---|---|---|---|---|---|---|
| * = frozen; +* = after 5 hours visible small crystals are formed in the solution, the solution does not freeze; + = after 5 hours the solution does not freeze and no visible small crystals are formed in the solution; A = a viscosity of < 20 mPas at -12° C; B = a viscosity of ≥ 20 mPas to < 25 mPas at -12° C; C = a viscosity of ≥ 25 mPas to ≤ 35 mPas at -12° C. | | | | | | | |

Table 2 shows that examples E1 to E2 having a good spraying performance up to -12° C and don't freeze at -15° C. Examples E3 freeze at -15° C. Examples E4 to E7 posses an excellent spraying performance at -15° C.

**Table 3: Teat Dip Formulations E8 to E13 of the invention**

| wt.-% based on the total composition | **E8** | **E9** | **E10** | **E11** | **E12** | **E13** |
|---|---|---|---|---|---|---|
| Dest. Water | | | | | | |
| Sorbitol | 0.4 | 1.6 | 1.8 | 0.4 | 1.6 | 1.8 |
| Glycerin | 15 | 15 | 16 | 15 | 15 | 16 |
| Propylene glycol | 5 | 20 | 6 | 5 | 20 | 6 |
| Isopropanol | 5 | 5 | 6 | 5 | 5 | 6 |
| Formic acid | 1 | 1 | 1 | | | |
| Octanoic acid | | | | 0.4 | 0.4 | 0.4 |
| freezing at -15° C | + | + | + | + | + | + |
| freezing at -12° C | + | + | + | + | + | + |
| freezing at -10° C | + | + | + | + | + | + |
| freezing at -8° C | + | + | + | + | + | + |
| visc. at -12° C /mPas (Brookfield; spdl. 61; 12 rpm) | A | A | A | A | A | A |

| | | | | | | |
|---|---|---|---|---|---|---|
| + = after 5 hours the solution does not freeze in total and no visible small crystals are formed in the solution A = a viscosity of < 20 mPas at -12° C; | | | | | | |

Table 3 shows that examples E8 to E13 posses an excellent spraying performance at - 15° C.

### Example 2 Spray Cone Angle and Spray Pattern Test

The composition of the inventions E1 to E13 was tested with respect to the spray cone angle and spray pattern.

The spray cone angle and spray pattern, i.e. the size of the moistened area as well as homogeneous spreading thereon increases with the decrease of viscosity of the composition to be sprayed.

The test was performed with a spray nozzle, type OpAd™, available at Afa Dispensing Group, whereby the spray nozzle opening was centrally arranged in a horizontal direction in a distance of 30 cm to a 625 cm² square plate.

Antimicrobial skin care liquid compositions according to the invention having at -12° C a viscosity of ≥ 25 mPas to ≤ 35 mPas, measured with a Brookfield viscometer, using a spindle 61 at 12 rpm, show a less preferred spray cone angle and spray pattern performance, due to the increased viscosity.

Antimicrobial skin care liquid compositions according to the invention having at -12° C a viscosity ≥ 20 mPas to < 25 mPas, measured with a Brookfield viscometer, using a spindle 61 at 12 rpm, show a more preferred spray cone angle and spray pattern performance, due to the low viscosity.

However, best results with respect to spray cone angle and spray pattern are achieved of ≥ 60° to ≤ 150° at -12° C with antimicrobial skin care liquid compositions according to the invention having a viscosity of < 20 mPas, preferably ≤ 15 mPas, and more preferred ≤ 10 mPas, measured with a Brookfield viscometer, using a spindle 61 at 12 rpm.

### Example 3 Sanitizing Efficacy of Teat Dip E7 against Mastitis Causing Organisms

The objective of the analysis was to determine the sanitizing efficacy of the teat dip composition 7 (from Table 2 above) against Staphylococcus aureus ATCC 6538, Escherichia coli ATCC 11229 and Streptococcus uberis ATCC 27958.

### TEST METHOD:

The method used in this example generally followed EN1656 (2009), in which the exposure contact time was 5 minutes, at 30°C. Aditionally, a skimmed milk solution was prepared with a final concentration of 1% skimmed milk (10g/l) in the test setup. In detail, 1 ml of this interfering substance is mixed with 1 ml of the selected bacterial test suspensions. After an incubation time of 2 min +/- 10 sec, 8.0 ml of the ready-to-use product test solution is added for the corresponding contact time of 5 min. Just before the end of the contact time, 1.0 ml of the test mixture is then transferred into a tube containing 8.0 ml of the neutralizer as given below and 1.0 ml of water. After the defined neutralization time, 1.0 ml of the mixture in duplicate is then used for the spread plate or pour plate technique to count measurable CFU numbers after an appropriate incubation time. The required controls are run in parallel. In order to claim bactericidal efficacy according to EN 1656, the product shall demonstrate at least a 4 decimal log (lg) reduction.

**Table 4**

| **METHOD PARAMETERS** | | | |
|---|---|---|---|
| Test Substance Name | Concentration | ml of Test Substance | ml of Diluent |
| E7 | 80% | 8 ml | 1 ml interfering substance + 1 ml water |

| | |
|---|---|
| **Test Systems:** | Staphylococcus aureus ATCC 6538, DSM 799 |
| | Escherichia coli ATCC 11229, DSM 682 |
| | Streptococcus uberis ATCC 27958, DSM 20569 |
| | |
| **Test Temperature:** | 30° C |
| **Exposure Time:** | 5 min. |
| **Neutralizer:** | Tween, Lecithin, Histidine, Na-thiosulfate |
| **Subculture Media:** | Tryptone Soya Agar (TSA) |
| **Incubation:** | 37° C for 48 hours |

### RESULTS:

**Table 5**

| Inoculum Numbers (CFU/mL) | |
|---|---|
| Organism | Log |
| *S. aureus* ATCC 6538 | 4.89 |
| *E. coli* ATCC 11229 | > 5 |
| *Streptococcus uberis* ATCC 27958 | > 5 |

**Table 6**

| Test Results | | | | |
|---|---|---|---|---|
| Test Substance | Test Organism | Survivors (CFU/ml) | Log Reduction | Percent Reduction |
| E7 | *S. aureus* | 160 | 4.89 | 99.999 |
| E7 | *E. coli* | 103 | > 5 | >99.999 |
| E7 | *S. uberis* | 50 | >5 | >99.999 |

### CONCLUSIONS :

Teat Dip Formulation E7 of table 6 achieved a > 99.999 percent reduction with a 5 min. exposure time at 30° C against Staphylococcus aureus ATCC 6538, Escherichia coli ATCC 11229 and Streptococcus uberis ATCC 27958. This percent reduction was unexpectedly high.

From the foregoing detailed description and examples, it will be evident that modifications and variations can be made to the compositions and methods of the invention.

## Claims

1. An antimicrobial skin care liquid composition, useful in cold temperature environments, comprising:
- ≥ 5 wt.-% to ≤ 25 wt.-% of glycerin;
- ≥ 0.1 wt.-% to ≤ 20 wt.-% of propylene glycol;
- ≥ 0.1 wt.-% to ≤ 20 wt.-% of at least one C₂ to C₆ organic monoalcohol selected from the group comprising ethanol, n-propanol, isopropanol, n-butanol, sec-butanol, isobutanol, tert-butanol, pentanol and/or hexanol;
- ≥ 0.1 wt.-% to ≤ 10 wt.-% of sorbitol; and
- ≥ 0.1 wt.-% to ≤ 15 wt.-% of at least one C2 to C6 organic acid; wherein
the weight-% of the components are based on the total weight amount of the antimicrobial skin care liquid composition; wherein the composition has a viscosity at -8° C, and/or at -20° C of ≥ 1 mPas to ≤ 35 mPas, measured with a Brookfield viscometer, using a spindle 61 at 12 rpm.

2. The antimicrobial skin care liquid composition of claim 1, wherein the composition comprises glycerin, propylene glycol, of at least one C₂ to C₆ organic monoalcohol selected from the group comprising ethanol, n-propanol, isopropanol, n-butanol, sec-butanol, iso-butanol, tert-butanol, pentanol and/or hexanol, sorbitol, and of at least one C2 to C6 organic acid, in a weight amount of ≥ 20 wt.-% and ≤ 80 wt.-%, preferably ≥ 25 wt.-% and ≤ 60 wt.-%, and more preferred ≥ 30 wt.-% and ≤ 50 wt.-%, based on the total weight amount of the antimicrobial skin care liquid composition.

3. The antimicrobial skin care liquid composition of claim 1 or 2, wherein the at least one C₂ to C₆ organic monoalcohol is selected from the group comprising n-propanol and/or isopropanol, wherein isopropanol is most preferred.

4. The antimicrobial skin care liquid composition of claims 1 to 3, wherein the antimicrobial skin care liquid composition comprises in addition water.

5. The antimicrobial skin care liquid composition of claims 1 to 4, wherein the antimicrobial skin care liquid composition comprises at least one C₁ to C₁₀ organic acid that is lactic acid.

6. The antimicrobial skin care liquid composition of claims 1 to 5, wherein the antimicrobial skin care liquid composition comprises at least one chlorite generating compound, preferably the chlorite generating compound is selected from the group of alkali and alkaline earth hypochlorites, and most preferred the chlorite generating compound is NaClO₂.

7. The antimicrobial skin care liquid composition of claims 1 to 6, wherein the antimicrobial skin care liquid composition comprises at least ≥ 15 wt.-% to ≤ 100 wt.-%, preferably ≥ 20 wt.-% to ≤ 90 wt.-%, further preferred ≥ 25 wt.-% to ≤ 80 wt.-%, also preferred ≥ 30 wt.-% to ≤ 70 wt.-%, in addition preferred ≥ 40 wt.-% to ≤ 70 wt.-%, and finally preferred ≥ 50 wt.-% to ≤ 60 wt.-%, of a mixture comprising:
- glycerin,
- propylene glycol,
- of at least one C₂ to C₆ organic monoalcohol selected from the group comprising ethanol, n-propanol, isopropanol, n-butanol, sec-butanol, iso-butanol, tert-butanol, pentanol and/or hexanol, and
- sorbitol.

8. The antimicrobial skin care liquid composition of claims 1 to 7, wherein the antimicrobial skin care liquid composition comprises at least ≥ 15 wt.-% to ≤ 100 wt.-%, preferably ≥ 20 wt.-% to ≤ 90 wt.-%, further preferred ≥ 25 wt.-% to ≤ 80 wt.-%, also preferred ≥ 30 wt.-% to ≤ 70 wt.-%, in addition preferred ≥ 40 wt.-% to ≤ 70 wt.-%, and finally preferred ≥ 50 wt.-% to ≤ 60 wt.-%, of a mixture comprising:
- glycerin;
- propyleneglycol;
- at least one C₂ to C₆ organic monoalcohol selected from the group comprising ethanol, n-propanol, isopropanol, n-butanol, sec-butanol, iso-butanol, tert-butanol, pentanol and/or hexanol, preferably n-propanol and/or isopropanol, wherein isopropanol is most preferred;
- sorbitol; and
- at least one C₂ to C₆ organic acid; wherein
the weight-% of the components are based on the total weight amount of the antimicrobial skin care liquid composition.

9. The antimicrobial skin care liquid composition of claims 1 to 8, wherein the antimicrobial skin care liquid composition has a freezing point ≤ -15° C and preferably has a freezing point ≤ -20° C.

10. The antimicrobial skin care liquid composition of claims 1 to 9, wherein the antimicrobial skin care liquid composition comprises in addition at least one alkali source, preferably selected from the group comprising alkali metal hydroxides, alkali metal salts, phosphates, amines, and mixtures thereof and more preferred triethanolamine.

11. The antimicrobial skin care liquid composition of claims 1 to 10, wherein the antimicrobial skin care liquid composition comprises in addition at least one hydrotrope, preferably the hydrotrope is selected from the group comprising aromatic hydrocarbon sulfonate, preferably xylene sulfonate, toluene sulfonate, cumene sulfonate, and/or n-octane sulfonate; or their sodium-, potassium- or ammonium salts or as salts of organic ammonium bases, and most preferred is cumene sulfonate.

12. The antimicrobial skin care liquid composition of claims 1 to 11, wherein the antimicrobial skin care liquid composition comprises in addition at least one surfactant, preferably an anionic surfactant, more preferred an C₆ to C₁₈ ether sulfate, and most preferred an lauryl ether sulfate with 2 EO.

13. The antimicrobial skin care liquid composition of claims 1 to 12, wherein the antimicrobial skin care liquid composition comprises:
- ≥ 5 wt.-% to ≤ 25 wt.-%, preferably ≥ 10 wt.-% to ≤ 20 wt.-%, and more preferred ≥ 13 wt.-% to ≤ 16 wt.-%, of glycerin;
- ≥ 0.1 wt.-% to ≤ 20 wt.-%, preferably ≥ 2 wt.-% to ≤ 10 wt.-%, and more preferred ≥ 3 wt.-% to ≤ 6 wt.-%, of propyleneglycol;
- ≥ 0.1 wt.-% to ≤ 20 wt.-%, preferably ≥ 2 wt.-% to ≤ 10 wt.-%, and more preferred ≥ 3 wt.-% to ≤ 6 wt.-%, of at least one C₂ to C₆ organic monoalcohol, preferably propanol and more preferred isopropanol;
- ≥ 0.1 wt.-% to ≤ 10 wt.-%, preferably ≥ 0.5 wt.-% to ≤ 5.0 wt.-%, and more preferred ≥ 1 wt.-% to ≤ 2.5 wt.-%, of sorbitol;
- ≥ 0 wt.-% to ≤ 5 wt.-%, preferably ≥ 0.1 wt.-% to ≤ 3 wt.-%, further preferred ≥ 0.5 wt.-% to ≤ 2 wt.-%, and more preferred ≥ 1 wt.-% to ≤ 1.5 wt.-%, of at least one surfactant, preferably an anionic surfactant, more preferred an C₆ to C₁₈ ether sulfate, and most preferred an lauryl ether sulfate with 2 EO;
- ≥ 0.1 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one C₂ to C₆ organic acid, preferably lactic acid;
- ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one alkali source, preferably triethanolamine;
- ≥ 0 wt.-% to ≤ 15 wt.-%, preferably ≥ 1 wt.-% to ≤ 12 wt.-%, further preferred ≥ 5 wt.-% to ≤ 10 wt.-%, and more preferred ≥ 6 wt.-% to ≤ 8 wt.-%, of at least one hydrotrope, preferably an aromatic hydrocarbon sulfonate, xylene sulfonate, toluene sulfonate, cumene sulfonate, and/or n-octane sulfonate and most preferred cumene sulfonate;
- ≥ 0 wt.-% to ≤ 30 wt.-%, preferably ≥ 0.01 wt.-% to ≤ 20 wt.-%, further preferred ≥ 1 wt.-% to ≤ 10 wt.-%, in addition preferred ≥ 3 wt.-% to ≤ 8 wt.-%, and more preferred ≥ 5 wt.-% to ≤ 7 wt.-%, of at least one chlorite generating compound, preferably an alkali and/or alkaline earth hypochlorite, and more preferred NaClO₂; and
- ≥ 0 wt.-% water, preferably the rest is water.

14. The antimicrobial skin care liquid composition of claims 1 to 13 for use in controlling mastitis in milk producing animals, the use comprising applying, preferably spraying, the antimicrobial skin care liquid composition to a teat of an animal.

## Patentansprüche

1. Flüssige antimikrobielle Hautpflegezusammensetzung, die für Niedertemperaturumgebungen geeignet ist, umfassend:
- ≥ 5 Gew.-% bis ≤ 25 Gew.-% Glycerin;
- ≥ 0,1 Gew.-% bis ≤ 20 Gew.-% Propylenglycol;
- ≥ 0,1 Gew.-% bis ≤ 20 Gew.-% mindestens eines organischen C₂- bis C₆-Monoalkohols, der ausgewählt ist aus der Gruppe bestehend aus Ethanol, n-Propanol, Isopropanol, n-Butanol, sek-Butanol, Isobutanol, tert-Butanol, Pentanol und/oder Hexanol;
- ≥ 0,1 Gew.-% bis ≤ 10 Gew.-% Sorbit; und;
- ≥ 0,1 Gew.-% bis ≤ 15 Gew.-% mindestens einer organischen C₂- bis C₆-Säure; wobei die Gewichts-% der Bestandteile auf dem Gesamtgewicht der flüssigen antimikrobiellen Hautpflegezusammensetzung basieren; wobei die Zusammensetzung eine Viskosität bei -8 °C und/oder bei -20 °C von ≥ 1 mPas bis ≤ 35 mPas aufweist, gemessen mit einem Brookfield-Viskosimeter unter Verwendung einer Spindel 61 bei 12 UpM.

2. Flüssige antimikrobielle Hautpflegezusammensetzung nach Anspruch 1, wobei die Zusammensetzung umfasst: Glycerin, Propylenglycol, mindestens einen organischen C₂- bis C₆-Monoalkohol, der ausgewählt ist aus der Gruppe bestehend aus Ethanol, n-Propanol, Isopropanol, n-Butanol, sek-Butanol, Isobutanol, tert-Butanol, Pentanol und/oder Hexanol, Sorbit und mindestens eine organische C₂- bis C₆-Säure in einem Gewichtsanteil von ≥ 20 Gew.-% und ≤ 80 Gew.-%, vorzugsweise ≥ 25 Gew.-% und ≤ 60 Gew.-%, und stärker bevorzugt ≥ 30 Gew.-% und ≤ 50 Gew.-%, basierend auf dem Gesamtgewicht der flüssigen antimikrobiellen Hautpflegezusammensetzung.

3. Flüssige antimikrobielle Hautpflegezusammensetzung nach Anspruch 1 oder 2, wobei der mindestens eine organische C₂- bis C₆-Monoalkohol ausgewählt ist aus der Gruppe bestehend aus n-Propanol und/oder Isopropanol, wobei Isopropanol am stärksten bevorzugt ist.

4. Flüssige antimikrobielle Hautpflegezusammensetzung nach einem der Ansprüche 1 bis 3, wobei die flüssige antimikrobielle Hautpflegezusammensetzung zusätzlich Wasser umfasst.

5. Flüssige antimikrobielle Hautpflegezusammensetzung nach einem der Ansprüche 1 bis 4, wobei die flüssige antimikrobielle Hautpflegezusammensetzung mindestens eine organische C₁- bis C₁₀-Säure umfasst, bei der es sich um Milchsäure handelt.

6. Flüssige antimikrobielle Hautpflegezusammensetzung nach einem der Ansprüche 1 bis 5, wobei die flüssige antimikrobielle Hautpflegezusammensetzung mindestens eine Chlorit-erzeugende Verbindung umfasst, wobei die Chlorit-erzeugende Verbindung vorzugsweise ausgewählt ist aus der Gruppe bestehend aus Alkali- und Erdalkalihypochloriten, und wobei die Chlorit-erzeugende Verbindung am stärksten bevorzugt NaClO₂ ist.

7. Flüssige antimikrobielle Hautpflegezusammensetzung nach einem der Ansprüche 1 bis 6, wobei die flüssige antimikrobielle Hautpflegezusammensetzung mindestens ≥ 15 Gew.-% bis ≤ 100 Gew.-%, bevorzugt ≥ 20 Gew.-% bis ≤ 90 Gew.-%, ferner bevorzugt ≥ 25 Gew.-% bis ≤ 80 Gew.-%, ebenfalls bevorzugt ≥ 30 Gew.-% bis ≤ 70 Gew.-%, außerdem bevorzugt ≥ 40 Gew.-% bis ≤ 70 Gew.-% und schließlich bevorzugt ≥ 50 Gew.-% bis ≤ 60 Gew.-% einer Mischung umfasst, die umfasst:
- Glycerin,
- Propylenglycol,
- mindestens einen organischen C₂- bis C₆-Monoalkohol, der ausgewählt ist aus der Gruppe bestehend aus Ethanol, n-Propanol, Isopropanol, n-Butanol, sek-Butanol, Isobutanol, tert-Butanol, Pentanol und/oder Hexanol, und
- Sorbit.

8. Flüssige antimikrobielle Hautpflegezusammensetzung nach einem der Ansprüche 1 bis 7, wobei die flüssige antimikrobielle Hautpflegezusammensetzung mindestens ≥ 15 Gew.-% bis ≤ 100 Gew.-%, bevorzugt ≥ 20 Gew.-% bis ≤ 90 Gew.-%, ferner bevorzugt ≥ 25 Gew.-% bis ≤ 80 Gew.-%, ebenfalls bevorzugt ≥ 30 Gew.-% bis ≤ 70 Gew.-%, außerdem bevorzugt ≥ 40 Gew.-% bis ≤ 70 Gew.-% und schließlich bevorzugt ≥ 50 Gew.-% bis ≤ 60 Gew.-% einer Mischung umfasst, die umfasst:
- Glycerin;
- Propylenglycol;
- mindestens einen organischen C₂- bis C₆-Monoalkohol, der ausgewählt ist aus der Gruppe bestehend aus Ethanol, n-Propanol, Isopropanol, n-Butanol, sek-Butanol, Isobutanol, tert-Butanol, Pentanol und/oder Hexanol, vorzugsweise n-Propanol und/oder Isopropanol, wobei Isopropanol am stärksten bevorzugt ist;
- Sorbit; und
- mindestens eine organische C₂- bis C₆-Säure; wobei
die Gew.-% der Bestandteile auf dem Gesamtgewicht der flüssigen antimikrobiellen Hautpflegezusammensetzung basieren.

9. Flüssige antimikrobielle Hautpflegezusammensetzung nach einem der Ansprüche 1 bis 8, wobei die flüssige antimikrobielle Hautpflegezusammensetzung einen Erstarrungspunkt von ≤ -15° C und vorzugsweise einen Erstarrungspunkt von ≤ -20° C aufweist.

10. Flüssige antimikrobielle Hautpflegezusammensetzung nach Anspruch 1 bis 9, wobei die flüssige antimikrobielle Hautpflegezusammensetzung zusätzlich mindestens eine Alkaliquelle umfasst, die vorzugsweise ausgewählt ist aus der Gruppe bestehend aus Alkalimetallhydroxiden, Alkalimetallsalzen, Phosphaten, Aminen und Mischungen davon, und am stärksten bevorzugt Triethanolamin.

11. Flüssige antimikrobielle Hautpflegezusammensetzung nach einem der Ansprüche 1 bis 10, wobei die flüssige antimikrobielle Hautpflegezusammensetzung zusätzlich mindestens ein Hydrotropikum umfasst, wobei das Hydrotropikum vorzugsweise ausgewählt ist aus der Gruppe bestehend aus aromatischem Kohlenwasserstoffsulfonat, vorzugsweise Xylolsulfonat, Toluolsulfonat, Cumolsulfonat und/oder n-Octansulfonat; oder deren Natrium-, Kalium- oder Ammoniumsalzen oder als Salze organischer Ammoniumbasen, und am stärksten bevorzugt Cumolsulfonat.

12. Flüssige antimikrobielle Hautpflegezusammensetzung nach einem der Ansprüche 1 bis 11, wobei die flüssige antimikrobielle Hautpflegezusammensetzung außerdem mindestens ein Tensid, vorzugsweise ein anionisches Tensid, stärker bevorzugt ein C₆- bis C₁₈-Ethersulfat und am stärksten bevorzugt ein Laurylethersulfat mit 2 EO umfasst.

13. Flüssige antimikrobielle Hautpflegezusammensetzung nach einem der Ansprüche 1 bis 12, wobei die flüssige antimikrobielle Hautpflegezusammensetzung umfasst:
- ≥ 5 Gew.-% bis ≤ 25 Gew.-%, bevorzugt ≥ 10 Gew.-% bis ≤ 20 Gew.-% und stärker bevorzugt ≥ 13 Gew.-% bis ≤ 16 Gew.-% Glycerin;
- ≥ 0,1 Gew.-% bis ≤ 20 Gew.-%, bevorzugt ≥ 2 Gew.-% bis ≤ 10 Gew.-% und stärker bevorzugt ≥ 3 Gew.-% bis ≤ 6 Gew.-% Propylenglycol;
- ≥ 0,1 Gew.-% bis ≤ 20 Gew.-%, bevorzugt ≥ 2 Gew.-% bis ≤ 10 Gew.-% und stärker bevorzugt ≥ 3 Gew.-% bis ≤ 6 Gew.-% mindestens eines organischen C₂- bis C₆-Monoalkohols, vorzugsweise Propanol und am stärksten bevorzugt Isopropanol;
- ≥ 0,1 Gew.-% bis ≤ 10 Gew.-%, bevorzugt ≥ 0,5 Gew.-% bis ≤ 5,0 Gew.-% und stärker bevorzugt ≥ 1 Gew.-% bis ≤ 2,5 Gew.-% Sorbit;
- ≥ 0 Gew.-% bis ≤ 5 Gew.-%, vorzugsweise ≥ 0,1 Gew.-% bis ≤ 3 Gew.-%, ferner bevorzugt ≥ 0,5 Gew.-% bis ≤ 2 Gew.-% und stärker bevorzugt ≥ 1 Gew.-% bis ≤ 1,5 Gew.-% mindestens eines Tensids, vorzugsweise eines anionischen Tensids, stärker bevorzugt eines C₆- C₁₈-Ethersulfats, und am stärksten bevorzugt Laurylethersulfat mit 2 EO;
- ≥ 0,1 Gew.-% bis ≤ 15 Gew.-%, vorzugsweise ≥ 1 Gew.-% bis ≤ 12 Gew.-%, ferner bevorzugt ≥ 5 Gew.-% bis ≤ 10 Gew.-% und am stärksten bevorzugt ≥ 6 Gew.-% bis ≤ 8 Gew.-% von mindestens einer organischen C₂- bis C₆-Säure, vorzugsweise Milchsäure;
- ≥ 0 Gew.-% bis ≤ 15 Gew.-%, vorzugsweise ≥ 1 Gew.-% bis ≤ 12 Gew.-%, ferner bevorzugt ≥ 5 Gew.-% bis ≤ 10 Gew.-% und stärker bevorzugt ≥ 6 Gew.-% bis ≤ 8 Gew.-% mindestens einer Alkaliquelle, vorzugsweise Triethanolamin;
- ≥ 0 Gew.-% bis ≤ 15 Gew.-%, vorzugsweise ≥ 1 Gew.-% bis ≤ 12 Gew.-%, ferner bevorzugt ≥ 5 Gew.-% bis ≤ 10 Gew.-% und stärker bevorzugt ≥ 6 Gew.-% bis ≤ 8 Gew.-% mindestens eines Hydrotropikums, vorzugsweise eines aromatischen Kohlenwasserstoffsulfonats, Xyolosulfonats, Toluolsulfonats, Cumolsulfonats und/oder n-Octansulfonats und am stärksten bevorzugt eines Cumolsulfonats;
- ≥ 0 Gew.-% bis ≤ 30 Gew.-%, vorzugsweise ≥ 0,01 Gew.-% bis ≤ 20 Gew.-%, ferner bevorzugt ≥ 1 Gew.-% bis ≤ 10 Gew.-%, zusätzlich bevorzugt ≥ 3 Gew.-% bis ≤ 8 Gew.-% und stärker bevorzugt ≥ 5 Gew.-% bis ≤ 7 Gew.-% mindestens einer Chloriterzeugenden Verbindung, vorzugsweise eines Alkali- oder Erdalkalihypochlorits, und stärker bevorzugt von NaClO₂; und
- ≥ 0 Gew-% Wasser, wobei der Rest vorzugsweise Wasser ist.

14. Flüssige antimikrobielle Hautpflegezusammensetzung nach einem der Ansprüche 1 bis 13 zur Verwendung bei der Eindämmung von Mastitis bei milchproduzierenden Tieren, wobei die Verwendung das Aufbringen, vorzugsweise das Aufsprühen, der flüssigen antimikrobiellen Hautpflegezusammensetzung auf ein Euter eines Tieres umfasst.

## Revendications

1. Composition liquide antimicrobienne pour le soin de la peau, utile dans des environnements à température froide, comprenant :
- ≥ 5 % en poids à ≤ 25 % en poids de glycérine ;
- ≥ 0,1 % en poids à ≤ 20 % en poids de propylène glycol ;
- ≥ 0,1 % en poids à ≤ 20 % en poids d'au moins un monoalcool organique C₂ à C₆ sélectionné dans le groupe comprenant l'éthanol, le n-propanol, l'isopropanol, le n-butanol, le sec-butanol, l'isobutanol, le tert-butanol, le pentanol et/ou l'hexanol ;
- ≥ 0,1 % en poids à ≤ 10% en poids de sorbitol ; et
- ≥ 0,1 % en poids à ≤ 15 % en poids d'au moins un acide organique C₂ à C₆ ; dans lequel le pourcentage en poids des composants est basé sur la quantité pondérale totale de la composition liquide antimicrobienne pour le soin de la peau ; dans laquelle la composition a une viscosité à -8°C, et/ou à -20°C de ≥ 1 mPas à ≤ 35 mPas, mesurée avec un viscosimètre Brookfield, en utilisant une broche 61 à 12 tours/min.

2. Composition liquide antimicrobienne pour le soin de la peau selon la revendication 1, dans laquelle la composition comprend de la glycérine, du propylène glycol, d'au moins un monoalcool organique C₂ à C₆ sélectionné dans le groupe comprenant l'éthanol, le n-propanol, l'isopropanol, le n-butanol, le sec-butanol, l'isobutanol, tert-butanol, le pentanol et/ou l'hexanol, le sorbitol, et d'au moins un acide organique C₂ à C₆, en une quantité pondérale de ≥ 20 % en poids et ≤ 80 % en poids, de préférence ≥ 25 % en poids et ≤ 60 % en poids, et de manière encore plus préférée ≥ 30 % en poids et ≤ 50 % en poids, sur la base de la quantité pondérale totale de la composition liquide antimicrobienne pour le soin de la peau.

3. Composition liquide antimicrobienne pour le soin de la peau selon la revendication 1 ou 2, dans laquelle le au moins un monoalcool organique C₂ à C₆ est sélectionné dans le groupe comprenant le n-propanol et/ou l'isopropanol, dans lequel l'isopropanol est le plus préféré.

4. Composition liquide antimicrobienne pour le soin de la peau selon les revendications 1 à 3, dans laquelle la composition liquide antimicrobienne pour le soin de la peau comprend en outre de l'eau.

5. Composition liquide antimicrobienne pour le soin de la peau selon les revendications 1 à 4, dans laquelle la composition liquide antimicrobienne pour le soin de la peau comprend au moins un acide organique C₁ à C₁₀ qui est de l'acide lactique.

6. Composition liquide antimicrobienne pour le soin de la peau selon les revendications 1 à 5, dans laquelle la composition liquide antimicrobienne pour le soin de la peau comprend au moins un composé générateur de chlorite, de préférence le chlorite est sélectionné dans le groupe des hypochlorites alcalins et alcalino-terreux, et de manière la plus préférée le composé est du NaClO₂.

7. Composition liquide antimicrobienne pour le soin de la peau selon les revendications 1 à 6, dans laquelle la composition liquide antimicrobienne pour le soin de la peau comprend au moins ≥ 15 % en poids à ≤ 100 % en poids, de préférence ≥ 20 % en poids à ≤ 90 % en poids, de manière plus préférée ≥ 25 % en poids à ≤ 80 % en poids, de manière plus préférée encore ≥ 30 % en poids à ≤ 70 % en poids, de manière encore plus préférée ≥ 40 % en poids à ≤ 70 % en poids, et de manière la plus préférée ≥ 50 % en poids à ≤ 60 % en poids, d'un mélange comprenant :
- de la glycérine,
- du propylène glycol,
- d'au moins un monoalcool organique C₂ à C₆ sélectionné dans le groupe comprenant l'éthanol, le n-propanol, l'isopropanol, le n-butanol, le sec-butanol, l'iso-butanol, le tert-butanol, le pentanol et/ou l'hexanol, et
- du sorbitol.

8. Composition liquide antimicrobienne pour le soin de la peau selon les revendications 1 à 7, dans laquelle la composition liquide antimicrobienne pour le soin de la peau comprend au moins ≥ 15 % en poids à ≤ 100 % en poids, de préférence ≥ 20 % en poids à ≤ 90 % en poids, de manière plus préférée ≥ 25 % en poids à ≤ 80 % en poids, de manière plus préférée encore ≥ 30 % en poids à ≤ 70 % en poids, de manière encore plus préférée ≥ 40 % en poids à ≤ 70 % en poids, et de manière la plus préférée ≥ 50 % en poids à ≤ 60 % en poids, d'un mélange comprenant :
- de la glycérine ;
- du propylène glycol ;
- au moins un monoalcool organique C₂ à C₆ sélectionné dans le groupe comprenant l'éthanol, le n-propanol, l'isopropanol, le n-butanol, le sec-butanol, l'isobutanol, le tert-butanol, le pentanol et / ou l'hexanol, de préférence le n-propanol et/ou l'isopropanol, dans lequel l'isopropanol est le plus préféré ;
- du sorbitol ; et
- au moins un acide organique C₂ à C₆ ; dans lequel
le pourcentage en poids des composants est basé sur la quantité pondérale totale de la composition liquide antimicrobienne pour le soin de la peau.

9. Composition liquide antimicrobienne pour le soin de la peau selon les revendications 1 à 8, dans laquelle la composition liquide antimicrobienne pour le soin de la peau a un point de congélation ≤ -15°C et de préférence un point de congélation ≤ -20°C.

10. Composition liquide antimicrobienne pour le soin de la peau selon les revendications 1 à 9, dans laquelle la composition liquide antimicrobienne pour le soin de la peau comprend en outre au moins une source alcaline, de préférence sélectionnée dans le groupe comprenant les hydroxydes de métaux alcalins, les sels de métaux alcalins, les phosphates, les amines et leurs mélanges, de manière encore plus préférée la triéthanolamine.

11. Composition liquide antimicrobienne pour le soin de la peau selon les revendications 1 à 10, dans laquelle la composition liquide antimicrobienne pour le soin de la peau comprenant en outre au moins un hydrotrope, de préférence, l'hydrotrope est sélectionné dans le groupe comprenant un sulfonate d'hydrocarbure aromatique, de préférence un sulfonate de xylène, un sulfonate de toluène, un sulfonate de cumène et/ou un sulfonate de n-octane ; ou leurs sels de sodium, de potassium ou d'ammonium ou sous forme de sels de bases d'ammonium organiques, et de manière la plus préférée est le sulfonate de cumène.

12. Composition liquide antimicrobienne pour le soin de la peau selon les revendications 1 à 11, dans laquelle la composition liquide antimicrobienne pour le soin de la peau comprend en outre au moins un agent tensioactif, de préférence un agent tensioactif anionique, de manière encore plus préférée un sulfate d'éther C₆ à C₁₈, et de manière la plus préférée un lauryl éther sulfate avec 2 OE.

13. Composition liquide antimicrobienne pour le soin de la peau selon les revendications 1 à 12, dans laquelle la composition liquide antimicrobienne pour le soin de la peau comprend :
- ≥ 5% en poids à ≤ 25 % en poids, de préférence ≥ 10 % en poids à ≤ 20 % en poids, et de manière encore plus préférée ≥ 13 % en poids à ≤ 16 % en poids, de glycérine ;
- ≥ 0,1 % en poids à ≤ 20 % en poids, de préférence ≥ 2 % en poids à ≤ 10 % en poids, et de manière encore plus préférée ≥ 3 % en poids à ≤ 6 % en poids, de propylèneglycol ;
- ≥ 0,1 % en poids à ≤ 20 % en poids, de préférence ≥ 2 % en poids à ≤ 10 % en poids, et de manière encore plus préférée ≥ 3 % en poids à ≤ 6 % en poids, d'au moins un monoalcool organique C₂ à C₆, de préférence du propanol et de manière encore plus préférée de l'isopropanol ;
- ≥ 0,1 % en poids à ≤ 10 % en poids, de préférence ≥ 0,5 % en poids à ≤ 5,0 % en poids, et de manière encore plus préférée ≥ 1 % en poids à ≤ 2,5 % en poids, de sorbitol ;
- ≥ 0 % en poids à ≤ 5 % en poids, de préférence ≥ 0,1 % en poids à ≤ 3% en poids, de manière plus préférée ≥ 0,5 % en poids à ≤ 2 % en poids, et de manière encore plus préférée ≥ 1 % en poids à ≤ 1,5 % en poids, d'au moins un agent tensioactif, de préférence un agent tensioactif anionique, de manière encore plus préférée un sulfate d'éther C₆ à C₁₈, et de manière la plus préférée un lauryl éther sulfate avec 2 OE ;
- ≥ 0,1 % en poids à ≤ 15 % en poids, de préférence ≥ 1 % en poids à ≤ 12 % en poids, de manière plus préférée ≥ 5 % en poids à ≤ 10 % en poids, et de manière encore plus préférée ≥ 6 % en poids à ≤ 8 % en poids d'au moins un acide organique C₂ à C₆, de préférence l'acide lactique ;
- ≥ 0 % en poids à ≤ 15 % en poids, de préférence ≥ 1 % en poids à ≤ 12 % en poids, de manière plus préférée ≥ 5 % en poids à ≤ 10 % en poids, et de manière encore plus préférée ≥ 6 % en poids à ≤ 8 % en poids, d'au moins une source alcaline, de préférence la triéthanolamine ;
- ≥ 0 % en poids à ≤ 15 % en poids, de préférence ≥ 1 % en poids à ≤ 12 % en poids, de manière plus préférée ≥ 5% en poids à ≤ 10 % en poids, et de manière encore plus préférée ≥ 6 % en poids à ≤ 8 % en poids, d'au moins un hydrotrope, de préférence un sulfonate d'hydrocarbure aromatique, un sulfonate de xylène, un sulfonate de toluène, un sulfonate de cumène et/ou un sulfonate de n-octane, et de manière la plus préférée, un sulfonate de cumène ;
- ≥ 0 % en poids à ≤ 30 % en poids, de préférence ≥ 0,01 % en poids à ≤ 20 % en poids, de manière plus préférée ≥ 1 % en poids à ≤ 10 % en poids, de manière plus préférée encore ≥ 3 % en poids à ≤ 8 % en poids, et de manière encore plus préférée ≥ 5 % en poids à ≤ 7 % en poids, d'au moins un composé générant du chlorite, de préférence un hypochlorite alcalin et/ou alcalino-terreux, et de manière encore plus préférée du NaClO₂ ; et
- ≥ 0 % en poids d'eau, de préférence le reste est de l'eau.

14. Composition liquide antimicrobienne pour le soin de la peau selon les revendications 1 à 13, destinée à être utilisée pour endiguer les mammites chez les animaux producteurs de lait, l'utilisation consistant à appliquer, de préférence à pulvériser, la composition liquide antimicrobienne pour le soin de la peau à un trayon d'un animal.
